# EUROPÄISCHE PATENTANMELDUNG

(11) **EP 4 588 889 A1**
(43) Veröffentlichungstag der Anmeldung: **23.07.2025**
(21) Anmeldenummer: 24020022.0
(22) Anmeldetag: 16.01.2024
(51) Int. Cl.: C01B 3/34, B01J 37/12, B01J 37/16, B01J 37/18, B01J 37/20, B01J 37/22, B01J 38/02, B01J 38/04, B01J 38/08, B01J 38/10, B01J 38/16, C01B 3/48, C01C 1/04, C07C 29/151

(54) **VERFAHREN UND ANLAGENVERBUND ZUR BEARBEITUNG UNTERSCHIEDLICHER REAKTIONSEINSÄTZE**

(71) Anmelder: Linde GmbH, 82049 Pullach (DE)
(72) Erfinder: Schwarzhuber, Josef, 82049 Pullach (DE); Reinke, Michael, 82049 Pullach (DE)
(74) Vertreter: Fischer, Werner

(57) **Zusammenfassung**

Es wird ein Verfahren (200) zur Bearbeitung unterschiedlicher Reaktionseinsätze in unterschiedlichen Anlagen oder Anlagenkomponenten (110-150) vorgeschlagen, wobei die Anlagen oder Anlagenkomponenten (110-150) jeweils in einem Anfahr- und/oder Abfahrbetrieb unter Einsatz einer Anfahr-und/oder Abfahreinrichtung (10) betrieben werden, und wobei für die unterschiedlichen Anlagen oder Anlagenkomponenten (110-150) dieselbe Anfahr- und/oder Abfahreinrichtung (10) verwendet wird. Ein Anlagenverbund (100) zur Durchführung des Verfahrens wird ebenfalls vorgeschlagen.

## Beschreibung

Die Erfindung betrifft ein Verfahren und einen Anlagenverbund zur Bearbeitung unterschiedlicher Reaktionseinsätze.

### Hintergrund

Chemieparks, beispielsweise am Standort von Raffinerien, Energiequellen und dergleichen, umfassen häufig eine Vielzahl von Anlagen zur Herstellung unterschiedlicher Verfahrensprodukte, beispielsweise zur Erzeugung von Wasserstoff durch Umsetzung von gasförmigen, festen oder flüssigen Kohlenwasserstoffquellen wie Erdgas, Naphtha oder Kohle mittels katalytischer Reformierung in unterschiedlichen Ausgestaltungen wie Dampfreformierung (engl. Steam Methane Reforming, SMR), autotherme Reformierung (engl. Autothermal Reforming, ATR) oder partielle Oxidation (engl. Partial Oxidation, POX). Entsprechende Anlagen können Vorreaktoren, beispielsweise sogenannte Prereformer, und Konditionier- und/oder Reinigungseinrichtungen für Edukte oder Produkte umfassen.

Solche Verfahren können ein sogenanntes Synthesegas, also ein Gasgemisch, das Wasserstoff sowie Kohlenmonoxid und/oder Kohlendioxid umfasst, liefern. Weitere Anlagen oder Anlagenkomponenten können vorgesehen sein, um beispielsweise mittels einer Wassergasshift (engl. Water Gas Shift, WGS) in Richtung Kohlendioxid und Wasserstoff oder in umgekehrter Richtung (engl. Reverse WGS, RWGS) die Gehalte an Wasserstoff, Kohlenmonoxid und Kohlendioxid in geeigneter Weise einzustellen. Diese können in Form einer Hoch-, Mittel- oder Niedertemperaturshift (engl. High Temperature Shift, HTS, Medium Temperature Shift, MTS und Low Temperature Shift, LTS) oder isothermer Shift (engl. Isothermal Shift, ITS) durchgeführt werden. Reiner oder im Wesentlichen reiner Wasserstoff kann aus einem entsprechenden Synthesegas z.B. durch eine Druckwechseladsorption (engl. Pressure Swing Adsorption, PSA) gewonnen werden.

An entsprechenden Standorten können ferner beispielsweise Anlagen zur Methanolsynthese oder Hydroformylierung vorhanden sein, die ein entsprechendes Synthesegas - bei der Hydroformylierung auch als Oxogas bezeichnet - zu weiteren Verfahrensvor- und/oder-endprodukten umsetzen können. Auch Ammoniakanlagen, die beispielsweise auf Grundlage des Haber-Bosch-Verfahrens oder seiner Varianten arbeiten, können vorgesehen sein. Gleiches gilt für die erwähnten Reinigungs- und Konditioniereinrichtungen wie Hydrier- und Entschwefelungsreaktoren.

Es besteht der Wunsch nach Integrationskonzepten für die unterschiedlichen Anlagen und Anlagenkomponenten, insbesondere um Redundanzen abzubauen, eine Gesamteffizienz zu verbessern und die Investitions- und Betriebskosten (engl. Capital Expenses, CAPEX und Operating Expenses, OPEX), beispielsweise auch durch reduzierte Ersatzteilhaltung, zu verbessern.

### Übersicht

Vor diesem Hintergrund werden ein Verfahren und eine Anlage zur Herstellung eines Verfahrensprodukts, insbesondere von Wasserstoff, mit den Merkmalen der unabhängigen Patentansprüche vorgeschlagen. Ausgestaltungen sind Gegenstand der abhängigen Patentansprüche und der nachfolgenden Beschreibung.

Das vorgeschlagene Verfahren zur Bearbeitung unterschiedlicher Reaktionseinsätze in unterschiedlichen Anlagen oder Anlagenkomponenten umfasst, dass die Anlagen oder Anlagenkomponenten jeweils in einem Anfahr- und/oder Abfahrbetrieb unter Einsatz einer Anfahr- und/oder Abfahreinrichtung betrieben werden, wobei für die unterschiedlichen Anlagen oder Anlagenkomponenten dieselbe Anfahr- und/oder Abfahreinrichtung verwendet wird. In jeder der Anlagen oder Anlagenkomponenten schließt sich an den Anfahrbetrieb ein Regelbetrieb mit beliebiger Dauer in zeitlicher Folge an. Nach dem Abfahrbetrieb werden die Anlagen üblicherweise abgeschaltet. Das Anfahren dient damit zur Inbetriebnahme, das Abfahren zur Außerbetriebnahme. Sowohl das Anfahren als auch das Abfahren benötigt eine bestimmte Zeit und erfolgt ggf. unter Verwendung anderer als im regulären Betrieb verwendeter Medien, Temperaturen und dergleichen. Ausgestaltungen der Erfindung können sich auf ein Anfahren, ein Abfahren oder beides erstrecken.

Eine Anfahr- und/oder Abfahreinrichtung wird in den hier vorgeschlagenen Ausgestaltungen also für mehrere Anfahr- und/oder Abfahrschritte unterschiedlicher Anlagen oder Anlagenkomponenten genutzt. Dadurch wird zwar die eine Anfahr- und/oder Abfahreinrichtung teurer, aber insgesamt günstiger als die Installation von mehreren individuellen Anfahr- und/oder Abfahreinrichtungen. Ein weiterer Vorteil einer gemeinsamen Anfahr- und/oder Abfahrausrüstung ist, dass diese Anfahr- und/oder Abfahreinrichtung dadurch öfter verwendet wird und damit zuverlässiger funktioniert, da nicht funktionierende Anlagenteile zuverlässiger entdeckt bzw. haltbarer ausgelegt werden können.

In bestimmten, hier vorgeschlagenen Ausgestaltungen umfasst der Anfahr- und/oder Abfahrbetrieb zumindest einer der Anlagen oder Anlagenkomponenten eine Behandlung eines Katalysators mit einem Behandlungsmittel, wobei das Behandlungsmittel unter Verwendung der Anfahr- und/oder Abfahreinrichtung bereitgestellt wird. Durch die Verwendung einer gemeinsamen Anfahr- und/oder Abfahreinrichtung kann insbesondere ein Behandlungsmittel höherer Reinheit, definierterer Zusammensetzung oder exakterer Temperatur bereitgestellt werden, da die Anfahr- und/oder Abfahreinrichtung beispielsweise mit genaueren und aufwendigeren Einstell- und Regelmitteln ausgestattet werden kann.

In bestimmten, hier vorgeschlagenen Ausgestaltungen umfasst die Behandlung des Katalysators eine Reduktion des Katalysators und das Behandlungsmittel ist ein Reduktionsmittel, das insbesondere aus Methan, Wasserstoff, Methanol, Ammoniak und Harnstoff oder Kombinationen hiervon ausgewählt ist. Die Behandlung kann auch eine Oxidation sein und das Behandlungsmittel ist ein Oxydationsmittel, das insbesondere aus Methan, Wasser, Kohlendioxid und Sauerstoff oder Kombinationen hiervon ausgewählt ist. Ein entsprechendes Behandlungsmittel kann auch eine Inertgaskomponente aufweisen, die aus Stickstoff, Helium, Neon und Argon oder Kombinationen hiervon ausgewählt ist.

In anderen, hier vorgeschlagenen Ausgestaltungen umfasst die Behandlung des Katalysators eine Erwärmung oder Kühlung des Katalysators und das Behandlungsmittel ist ein Temperiermittel, das insbesondere aus Dampf, Luft, einem Inertgas oder Kombinationen hiervon ausgewählt ist. Für beide Aspekte gelten die obigen Erläuterungen in besonderer Weise.

In bestimmten, hier vorgeschlagenen Ausgestaltungen umfasst der Anfahr- und/oder Abfahrbetrieb eine Druckbeaufschlagung und die Anfahr- und/oder Abfahreinrichtung weist eine oder mehrere Verdichtungseinrichtungen auf, oder der Anfahr- und/oder Abfahrbetrieb umfasst einen Abscheidebetrieb und die Anfahr- und/oder Abfahreinrichtung weist eine oder mehrere Abscheideeinrichtungen auf. Besonders wartungsintensive Apparate wie Verdichter können in den hier vorgeschlagenen Ausgestaltungen häufiger gewartet oder zuverlässiger ausgeführt werden, wie bereits oben erläutert.

In bestimmten, hier vorgeschlagenen Ausgestaltungen umfassen die Anlagen oder Anlagenkomponenten zumindest eine Anlage oder Anlagenkomponente, die aus einer Ammoniaksyntheseeinheit, einer Methanolsyntheseeinheit, einer Hochtemperaturshifteinheit, einer Mitteltemperaturshifteinheit, einer Niedertemperaturshifteinheit, einer isothermen Shifteinheit, einer Hydriereinheit, einer Prereformiereinheit, einer Entschwfelungseinheit, einer Dechlorierungseinheit, einer Quecksilberadsorptionseinheit, einer Methanisierungseinheit oder beliebigen Kombinationen hiervon ausgewählt ist. Die entsprechenden Anlagen oder Anlagenkomponenten werden unten detailliert erläutert.

In bestimmten, hier vorgeschlagenen Ausgestaltungen beträgt eine Anzahl der Anlagen oder Anlagenkomponenten 2, 3, 4, 5 oder mehr als 5 und insbesondere bis zu 10. Die vorgeschlagenen Ausgestaltungen eignen sich damit für eine Vielzahl von Anlagen oder Anlagenkomponenten, die grundsätzlich nicht begrenzt ist.

In bestimmten, hier vorgeschlagenen Ausgestaltungen ist vorgesehen, dass die Anfahr- und/oder Abfahreinrichtung in aufeinanderfolgenden oder disjunkten Zeiträumen für den Betrieb der Anlagen oder Anlagenkomponenten verwendet wird. In Zwischenzeiträumen ist beispielsweise jeweils eine Inspektion oder Wartung möglich, ohne den Betrieb der Anlagen oder Anlagenkomponenten zu beeinflussen.

Der vorgeschlagene Anlagenverbund ist zur Bearbeitung unterschiedlicher Reaktionseinsätze eingerichtet und weist unterschiedliche Anlagen oder Anlagenkomponenten auf, wobei die Anlagen oder Anlagenkomponenten jeweils für einen Anfahr- und/oder Abfahrbetrieb unter Einsatz einer Anfahr- und/oder Abfahreinrichtung eingerichtet sind, und wobei für die unterschiedlichen Anlagen oder Anlagenkomponenten dieselbe Anfahr- und/oder Abfahreinrichtung bereitgestellt ist.

Zu weiteren Merkmalen und Vorteilen eines entsprechenden Anlagenverbunds und Ausgestaltungen hiervon sei auf die obigen Erläuterungen betreffend das vorgeschlagene Verfahren und seine Ausgestaltungen ausdrücklich verwiesen, da diese hierfür in gleicher Weise gelten.

Entsprechendes gilt auch für einen Anlagenverbund, der dazu eingerichtet sein kann, ein Verfahren gemäß einer beliebigen Ausgestaltung durchzuführen.

### Zeichnungen

Ausführungsformen der Erfindung werden nachfolgend rein beispielhaft unter Bezugnahme auf die beigefügte Zeichnung beschrieben, wobei
Figur 1 einen Anlagenverbund gemäß einer Ausgestaltung veranschaulicht; und
Figur 2 ein Verfahren gemäß einer Ausgestaltung veranschaulicht.

### Ausführungsformen

Die nachfolgend beschriebenen Ausführungsformen werden lediglich zu dem Zweck beschrieben, den Leser beim Verständnis der beanspruchten und zuvor erläuterten Merkmale zu unterstützen. Sie stellen lediglich repräsentative Beispiele dar und sollen hinsichtlich der Merkmale der Erfindung nicht abschließend und/oder beschränkend betrachtet werden. Es versteht sich, dass die zuvor und nachfolgend beschriebenen Vorteile, Ausführungsformen, Beispiele, Funktionen, Merkmale, Strukturen und/oder anderen Aspekte nicht als Beschränkungen des Umfangs der Erfindung, wie er in den Ansprüchen definiert ist, oder als Beschränkungen von Äquivalenten zu den Ansprüchen zu betrachten sind, und dass andere Ausführungsformen verwendet und Änderungen vorgenommen werden können, ohne vom Umfang der beanspruchten Erfindung abzuweichen.

Unterschiedliche Ausführungsformen der Erfindung können weitere zweckmäßige Kombinationen der beschriebenen Elemente, Komponenten, Merkmale, Teile, Schritte, Mittel usw. umfassen, aufweisen, aus ihnen bestehen oder im Wesentlichen aus ihnen bestehen, auch wenn solche Kombinationen hier nicht spezifisch beschrieben sind. Darüber hinaus kann die Offenbarung andere Erfindungen umfassen, die gegenwärtig nicht beansprucht sind, die aber in Zukunft beansprucht werden können, insbesondere wenn sie vom Umfang der unabhängigen Ansprüche umfasst sind.

Erläuterungen, die sich auf Vorrichtungen, Apparate, Anordnungen, Systeme usw. gemäß Ausführungsformen der vorliegenden Erfindung beziehen, können auch für Verfahren, Prozesse, Methoden usw. gemäß den Ausführungsformen der vorliegenden Erfindung gelten und umgekehrt. Gleiche, gleich wirkende, in ihrer Funktion einander entsprechende, baulich identisch oder vergleichbar aufgebaute Elemente, Verfahrensschritte usw. können mit identischen Bezugszeichen angegeben sein.

Die nachfolgenden Erläuterungen und Definitionen, die einige Grundlagen der Erfindung betreffen, können für alle oder einen Teil der hier vorgestellten Ausgestaltungen gelten, und die Erläuterung bestimmter Aspekte im Zusammenhang mit nur einem Teil oder einer der Ausgestaltungen soll nicht dahingehend verstanden werden, dass diese Aspekte nicht auch mit anderen oder allen Ausgestaltungen, soweit technisch möglich und sinnvoll, verwirklicht sein können.

Sämtliche hier verwendeten Prozentangaben können sich auf Mol-, Mengen- oder Volumenanteile beziehen. Druckangaben in bar sind, soweit nicht anders erläutert, insbesondere als Absolutdrücke zu verstehen.

Die Konjunktion "und/oder" soll, wenn in einer Aufzählung vor dem letzten Begriff der Aufzählung verwendet, so verstanden werden, dass alle in der Aufzählung zuvor genannten Begriffe in beliebiger Weise miteinander kombiniert werden können. Mit anderen Worten ist mit "A, B und/oder C" "A und/oder B und/oder C" oder "wenigstens eines der Elemente A, B und C in beliebiger Kombination" gemeint.

Ist vorliegend von einem "Teil" eines Stoffstroms die Rede, kann es sich hierbei um einen Mengenanteil mit gleicher Zusammensetzung handeln, der von einem Ausgangsstrom lediglich abgezweigt wurde, aber auch um einen Anteil abweichender Zusammensetzung und ggf. nur eine Komponente des Ausgangsstroms, der mittels eines Verfahrens wie Kondensation, Evaporation, Sieden, Destillieren, Rektifizieren, Absorbieren, Adsorbieren, Flashen, Membrantrennen, Abscheiden oder dergleichen gebildet wird oder bei einem entsprechenden Schritt als Rest verbleibt. Ein "Teil" kann auch nach einer Kombination beliebiger der vorstehend benannten Schritte vorliegen, beispielsweise nach trenntechnischer Bearbeitung abgezweigten Anteils.

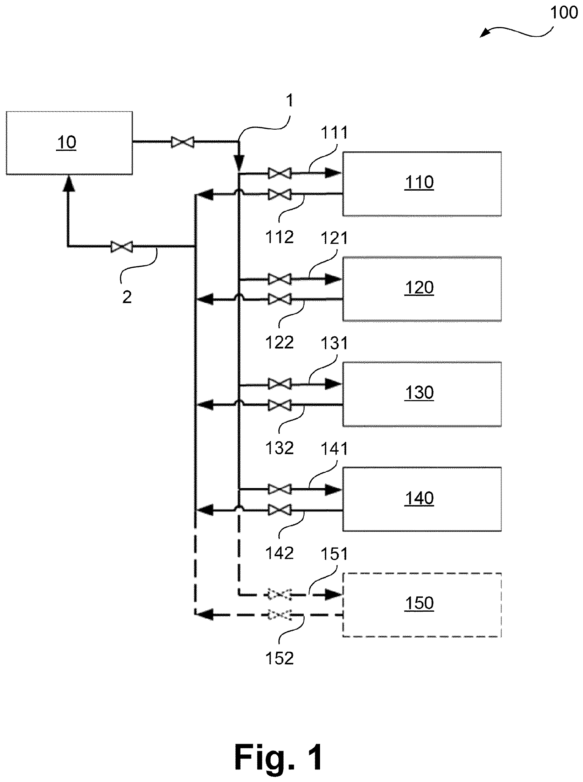

In Figur 1 ist zunächst ein Anlagenverbund gemäß einer hier vorgeschlagenen Ausgestaltung anhand eines stark vereinfachten Blockdiagramms schematisch veranschaulicht und insgesamt mit 100 bezeichnet.

In dem in Figur 1 veranschaulichten Anlagenverbund 100 sind dabei eine Reihe von Anlagen oder Anlagenkomponenten 110 bis 150 dargestellt, wobei die Anlage oder Anlagenkomponente 150 zur Veranschaulichung der generellen Erweiterbarkeit auf weitere Anlagen oder Anlagenkomponenten und der Möglichkeit, bestimmte Anlagen oder Anlagenkomponenten wegzulassen, gestrichelt dargestellt ist.

Die vorgeschlagenen Anlagenverbünde 100 und ihre jeweiligen Ausgestaltungen sind durch die Anzahl und Art der jeweiligen Anlagen oder Anlagenkomponenten 110 bis 150 und der dort jeweils durchgeführten Prozesse oder Teilprozesse sowie hinsichtlich der verarbeiteten Komponenten in keiner Weise beschränkt.

Nachfolgend sollen rein beispielhaft, und ohne die Erfindung hierdurch in irgendeiner Weise beschränken zu wollen, ausgewählte Anlagen und Anlagenkomponenten 110 bis 150, die Teil von hier vorgeschlagenen Ausgestaltungen sein können, im Hinblick auf die vorliegend vorgeschlagenen Maßnahmen erläutert werden.

Bei einer der Anlagen oder Anlagenkomponenten 110 bis 150 kann es sich beispielsweise um eine Einrichtung zur Herstellung von Synthesegas handeln. Zum Begriff "Synthesegas" und die hierzu verwendeten Prozesse sei auf die eingangs vorgenommenen Erläuterungen verwiesen. Nachfolgend wird für entsprechende Prozesse auch der Begriff "Reformierung" verwendet, worunter beispielsweise die Dampfreformierung, die autotherme Reformierung oder die Trockenreformierung bzw. Kohlendioxidreformierung fallen kann.

Weitere Prozesse, wie die partielle Oxidation, können ebenfalls in Ausgestaltungen der Erfindung zur Herstellung von Synthesegas verwendet werden, auch wenn sich die nachfolgenden Erläuterungen nicht spezifisch hierauf konzentrieren. Die Dampfreformierung wird nachfolgend als Beispielprozess vorgestellt, wobei die entsprechenden Erläuterungen aber auch für andere Verfahren zur Herstellung von Synthesegas gelten können.

Selbstverständlich gelten die entsprechenden Erläuterungen auch für die Herstellung von Wasserstoff oder Folgeverbindungen, die unter Einsatz von Synthesegas hergestellt werden können, wie beispielsweise Methanol oder Produkte einer Oxosynthese, und beliebige Zwischenschritte.

Bei der Dampfreformierung wird, wie in der US 10 294 102 B2 und in der allgemeinen Fachliteratur beschrieben, ein Reformierreaktor ("Ofen") mit zahlreichen mit Katalysator befüllten Reaktionsrohren ("Reformerrohren") verwendet, wobei die Reaktionsrohre typischerweise in parallelen Reihen angeordnet sind und in diesen die endotherme Dampfreformierungsreaktion abläuft. Wenn der Reformierreaktor in Betrieb genommen wird, nachdem der Katalysator in den Rohren ausgetauscht wurde, muss der Katalysator in den Reaktionsrohren von seinem ursprünglichen oxidierten Zustand in einen reduzierten Zustand überführt werden.

Die Reduktion des Dampfreformierungskatalysators kann durch die Einleitung von Dampf und eines Reduktionsmittels in den Katalysator durchgeführt werden. An vielen Standorten wird als Reduktionsmittel Erdgas (d.h. Methan) verwendet, das im sich anschließenden Normalbetrieb das primäre Ausgangsmaterial für die Dampfreformierung darstellt. Alternativ kann als Reduktionsmittel auch Wasserstoff verwendet werden, der beispielsweise von einer Wasserstoffproduktionsanlage oder aus einer bestehenden Pipeline herangeführt oder in flüssiger Form über Tanks und/oder Tanklaster geliefert werden kann. Ist die Reduktion des Dampfreformierungskatalysators auf diese Weise bzw. unter Einsatz dieser Reduktionsmittel nicht möglich oder erwünscht, kann bzw. können auch ein oder mehrere Verfahrensprodukte anderer Prozesse zum Einsatz kommen, z.B. Methanol, Ammoniak oder Harnstoff.

An dieser Stelle sei angemerkt, dass auch andere Katalysatoren für andere der Anlagen oder Anlagenkomponenten 110 bis 150 eine gleiche, vergleichbare oder abweichende Anfangs-/Endbehandlung in einem An-/Abfahrbetrieb erfordern können. Diese Anfangs-/Endbehandlung kann in einem einfachen Fall auch lediglich ein Ausspülen von Luft oder anderen Gasen aus dem Katalysator oder eine Erwärmung auf eine Anspringtemperatur des Katalysators, ab der dieser katalytisch aktiv ist, umfassen. Auch weitere Schritte wie Kalzinierung oder die erwähnte Katalysatorreduktion kann in bestimmten Fällen in der Anlage oder Anlagenkomponente 110 bis 150 erfolgen.

Die Reduzierung von Katalysatoren wird beispielsweise bei Anlagen oder Anlagenkomponenten 110 bis 150 zur Ammoniaksynthese, Methanolsynthese, Wassergasshift, insbesondere einer Mitteltemperaturshift, Niedertemperaturshift und Isothermalshift, Prereformierung und zur Feinentschwefelung eingesetzt, ist aber, wie entsprechende Ausgestaltungen, die hier vorgeschlagen werden, nicht hierauf beschränkt. Die Erwärmung von Katalysatoren wird beispielsweise bei Anlagen oder Anlagenkomponenten 110 bis 150 zur Prereformierung, Entschwefelung, Hochtemperaturshift und Ammoniaksynthese eingesetzt, ist aber ebenfalls nicht hierauf beschränkt.

Bei einer der Anlagen oder Anlagenkomponenten 110 bis 150 kann es sich auch um eine Anlage oder Anlagenkomponente 110 bis 150 zur Umsetzung von Zinkoxid und Schwefelwasserstoff zu Zinksulfid und Wasser bei ca. 350 °C handeln.

Ein Prereformer, der ebenfalls eine der Anlagen oder Anlagenkomponenten 110 bis 150 darstellen kann, kann beispielsweise in Form eines adiabat betriebenen katalytischen Reaktors bereitgestellt sein und dazu verwendet werden, in einem Einsatz wie Erdgas enthaltene höhere Kohlenwasserstoffe in Methan umzuwandeln. Dadurch kann eine Verkokung insbesondere in der Vorwärmung des Reformereinsatzes und am Eingang der Reaktionsrohre verhindert werden.

Ein oder mehrere Reaktoren zur Durchführung einer Wassergasshift in einer oder in beiden Richtungen können ebenfalls als eine oder mehrere der Anlagen oder Anlagenkomponenten 110 bis 150 vorgesehen sein. Wie ebenfalls bereits angesprochen, kann in entsprechenden Prozessen eine Hoch-, Mittel- und Niedertemperaturshift eingesetzt werden. Der Grund liegt darin, dass sich bei steigender Temperatur das chemische Gleichgewicht von den Reaktionsprodukten hin zu den Reaktionsedukten verschiebt. Bei höherer Temperatur liegt eine schnelle Kinetik, aber ein ungünstiges chemisches Gleichgewicht vor. Durch die Kombination der Temperaturstufen kann eine weitgehende Umsetzung erreicht werden. Auch der Einsatz einer Isothermalshift kann vorgesehen sein.

Die Methanisierung ist eine chemische Reaktion, bei der Kohlenstoffmonoxid oder Kohlenstoffdioxid in Methan umgewandelt wird. Bei dieser Reaktion reagiert Kohlenstoffmonoxid oder Kohlenstoffdioxid bei Temperaturen von 300 bis 700 °C mit Wasserstoff zu Methan und Wasser. Die Reaktion ist exotherm, wird jedoch durch einen Katalysator beschleunigt. Auch ein oder mehrere Reaktoren zur Methanisierung können als Anlagen oder Anlagenkomponenten 110 bis 150 bereitgestellt sein und Katalysatoren umfassen, die entsprechend behandelt werden können.

Der hier beispielhaft vorgestellte Anlagenverbund 100 ist also zur Bearbeitung unterschiedlicher Reaktionseinsätze in unterschiedlichen Anlagen oder Anlagenkomponenten 110 bis 150 eingerichtet, wobei die Anlagen oder Anlagenkomponenten 110 bis 150 jeweils in einem Anfahr- und/oder Abfahrbetrieb betrieben werden. Für den Anfahr- und/oder Abfahrbetrieb wird eine hier mit 10 bezeichnete, gemeinsame Anfahr- und/oder Abfahreinrichtung 10 bereitgestellt, die in den hier vorgeschlagenen Ausgestaltungen für die unterschiedlichen Anlagen oder Anlagenkomponenten 110 bis 150 dieselbe ist. Selbstverständlich schließt dies die Verwendung weiterer Anfahr- und/oder Abfahreinrichtungen, zusätzlich zu der dargestellten gemeinsamen Anfahr- und/oder Abfahreinrichtung 10, nicht aus.

In der hier veranschaulichten Ausgestaltung des Anlagenverbunds 100 wird mittels der Anfahr- und/oder Abfahreinrichtung über ein nicht gesondert bezeichnetes Ventil ein Medienstrom 1 bereitgestellt, bei dem es sich um ein Behandlungsmittel, insbesondere ein Reduktionsmittel für einen Katalysator, aber auch beispielsweise ein Temperierfluid zum Kühlen oder Erwärmen der Anlagen oder Anlagenkomponenten 110 bis 150 oder eines oder mehrerer Katalysatoren hierin, oder ein Druckfluid zur Druckbeaufschlagung handeln kann. Auch kann es sich bei dem Medienstrom 1 beispielsweise um eine Fraktion einer Abscheidung handeln, die in der Anfahr- und/oder Abfahreinrichtung 10 vorgenommen wird.

Mit anderen Worten kann ein Anfahr- und/oder Abfahrbetrieb zumindest einer der Anlagen oder Anlagenkomponenten 110 bis 150 in den hier betrachteten Varianten insbesondere eine Behandlung eines Katalysators mit einem Behandlungsmittel 1 umfassen, wobei das Behandlungsmittel 1 unter Verwendung der Anfahr- und/oder Abfahreinrichtung 10 bereitgestellt wird. Die Behandlung des Katalysators kann eine Reduktion des Katalysators umfassen und das Behandlungsmittel 1 kann ein Reduktionsmittel sein, das insbesondere aus Methan, Wasserstoff, Methanol, Ammoniak und Harnstoff oder Kombinationen hiervon ausgewählt sein kann. Die Behandlung des Katalysators eine Erwärmung oder Kühlung des Katalysators umfassen und das Behandlungsmittel 1 kann ein Temperiermittel sein, das insbesondere aus Dampf, Luft, einem Inertgas oder Kombinationen hiervon ausgewählt ist. Eine Vermischung von Reduktionsmittel und Temperiermittel ist auch möglich, um Reduktion und Anwärmung gleichzeitig ablaufen zu lassen.

Umfasst der Anfahr- und/oder Abfahrbetrieb beispielsweise eine Erwärmung oder Kühlung, kann die Anfahr- und/oder Abfahreinrichtung 10 einen oder mehrere elektrische oder nichtelektrische Heizer, Brenner, Wärmetauscher oder dergleichen aufweisen. Umfasst diese eine Druckbeaufschlagung, kann die Anfahr- und/oder Abfahreinrichtung 10 eine oder mehrere Verdichtungseinrichtungen aufweisen. Umfasst der Anfahr- und/oder Abfahrbetrieb einen Abscheidebetrieb, kann die Anfahr- und/oder Abfahreinrichtung 10 eine oder mehrere Abscheideeinrichtungen aufweisen.

Die jeweiligen Anlagen oder Anlagenkomponenten 110 bis 150 können in ihrem jeweiligen Anfahr- und/oder Abfahrbetrieb mit dem Behandlungsmittel 1 bzw. einem anderen Medium der erläuterten Arten versorgt werden. Dies kann über ebenfalls nicht gesondert bezeichnete Ventile in Einspeiseleitungen 111, 121, 131, 141 und 151 erfolgen. Entsprechend kann über nicht gesondert bezeichnete Ventile in Entnahmeleitungen 112, 122, 132, 142 und 152 eine Entnahme aus den Anlagen oder Anlagenkomponenten 110 bis 150 erfolgen. Auf diese Weise kann beispielsweise ein stofflich veränderter oder abgekühlter oder erwärmter Rückführstrom 2 in die Anfahr- und/oder Abfahreinrichtung 10 rückgeführt werden.

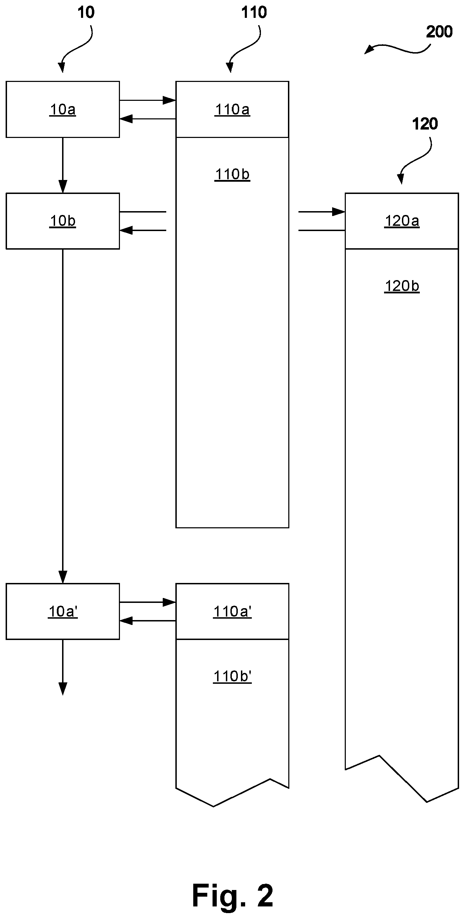

In Figur 2 ist ein Verfahren 200 gemäß einer hier vorgeschlagenen Ausgestaltung veranschaulicht, wobei eine Zeitachse senkrecht von oben nach unten in der Papierebene verläuft. Die Zeitachse kann beliebig nach unten verlängert sein. Der Einfachheit halber wird lediglich der Anfahrbetrieb zweier Anlagen oder Anlagenkomponenten 110 und 120 veranschaulicht.

Die in den entsprechenden Anlagen oder Anlagenkomponenten 110 und 120 realisierten Verfahrensmodi, nämlich ein Anfahrbetrieb und ein Regelbetrieb, der sich an den Anfahrbetrieb anschließt, sind jeweils mit nachgestellten Kleinbuchstaben a und b dargestellt, so dass der Anfahrbetrieb der Anlage oder Anlagenkomponente 110 mit 110a und der Regelbetrieb derselben mit 110b sowie der Anfahrbetrieb der Anlage oder Anlagenkomponente 120 mit 120a und der Regelbetrieb mit 120b bezeichnet ist.

In einem Schritt 10a wird die Anfahr- und/oderAbfahreinrichtung 10 für den Anfahrbetrieb 110a der Anlage oder Anlagekomponente 110 verwendet, die daran anschließend insbesondere ohne Verwendung der Anfahr- und/oder Abfahreinrichtung 10 im Regelbetrieb 110b weiterläuft. In einem Schritt 10b wird die Anfahr- und/oder Abfahreinrichtung 10 dann für den Anfahrbetrieb 120a der Anlage oder Anlagekomponente 120 verwendet, die daran anschließend ebenfalls insbesondere ohne Verwendung der Anfahr- und/oder Abfahreinrichtung 10 im Regelbetrieb 120b weiterläuft. Der Betrieb weiterer Anlagen oder Anlagenkomponenten 120 bis 150 kann in entsprechender Weise erfolgen.

Bei einem späteren Stopp des Regelbetriebs 110b bzw. 120b, der ein Wiederanfahren erfordert, kann die Anfahr- und/oder Abfahreinrichtung 10 erneut zum Einsatz kommen. Dies ist hier für die Anlage oder Anlagenkomponente 110 (mit einem aus Anschaulichkeitsgründen stark verkürzt dargestellten Regelbetrieb 11 0b) mit dem weiteren Anfahrbetrieb 110a' dargestellt, der mit Unterstützung der Anfahr- und/oder Abfahreinrichtung 10 in einem Schritt 10' erfolgt, und dem sich ein weiterer Regelbetrieb 110b' anschließt. Entsprechendes kann auch für die weiteren Anlagen oder Anlagenkomponenten 120 bis 150 gelten, wobei beliebige Zeiträume für Anfahr- und Regelbetriebe 110a, 120a, 110a', 110b und 120b vorgesehen sein können.

Die Anfahr- und/oder Abfahreinrichtung 10 kann also in den hier veranschaulichten Ausgestaltungen in aufeinanderfolgenden oder disjunkten Zeiträumen für den Betrieb der Anlagen oder Anlagenkomponenten 110 bis 150 verwendet werden.

## Patentansprüche

1. Verfahren (200) zur Bearbeitung unterschiedlicher Reaktionseinsätze in unterschiedlichen Anlagen oder Anlagenkomponenten (110-150), wobei die Anlagen oder Anlagenkomponenten (110-150) jeweils in einem Anfahr- und/oder Abfahrbetrieb unter Einsatz einer Anfahr- und/oder Abfahreinrichtung (10) betrieben werden, und wobei für die unterschiedlichen Anlagen oder Anlagenkomponenten (110-150) dieselbe Anfahr- und/oder Abfahreinrichtung (10) verwendet wird.

2. Verfahren (200) nach Anspruch 1, bei dem der Anfahr- oder Abfahrbetrieb zumindest einer der Anlagen oder Anlagenkomponenten (110-150) eine Behandlung eines Katalysators mit einem Behandlungsmittel umfasst, wobei das Behandlungsmittel unter Verwendung der Anfahr- und/oder Abfahreinrichtung (10) bereitgestellt wird.

3. Verfahren (200) nach Anspruch 2, bei dem die Behandlung des Katalysators eine Reduktion oder Oxidation des Katalysators umfasst und das Behandlungsmittel ein Reduktions- oder Oxidationsmittel ist.

4. Verfahren (200) nach Anspruch 3, bei dem das Behandlungsmittel für die Reduktion aus Methan, Wasserstoff, Methanol, Ammoniak und Harnstoff und für die Oxidation aus Wasser, Kohlendioxid und Sauerstoff oder Kombinationen hiervon ausgewählt ist.

5. Verfahren (200) nach Anspruch 3 oder 4, bei dem das Behandlungsmittel zumindest eine Inertgaskomponente aufweist, die insbesondere aus Stickstoff, Helium, Neon und Argon oder Kombinationen hiervon ausgewählt ist.

6. Verfahren (200) nach einem der Ansprüche 2 bis 5, bei dem die Behandlung des Katalysators eine Erwärmung oder Kühlung des Katalysators umfasst und das Behandlungsmittel ein Temperiermittel ist, das insbesondere aus Dampf, Luft, einem Inertgas oder Kombinationen hiervon ausgewählt ist.

7. Verfahren (200) nach einem der vorstehenden Ansprüche, bei dem der Anfahrbetrieb eine Druckbeaufschlagung umfasst und die Anfahr- und/oder Abfahreinrichtung (10) eine oder mehrere Verdichtungseinrichtungen aufweist.

8. Verfahren (200) nach einem der vorstehenden Ansprüche, bei dem der Anfahr- und/oder Abfahrbetrieb einen Abscheidebetrieb umfasst und die Anfahr- und/oder Abfahreinrichtung (10) eine oder mehrere Abscheideeinrichtungen aufweist.

9. Verfahren (200) nach einem der vorstehenden Ansprüche, bei dem die Anlagen oder Anlagenkomponenten (110-150) eine oder mehrere Anlagen oder Anlagenkomponenten (110-150) sind oder umfassen, die aus einer Ammoniaksyntheseeinheit, einer Methanolsyntheseeinheit, einer Hochtemperaturshifteinheit, einer Mitteltemperaturshifteinheit, einer Niedertemperaturshifteinheit, einer isothermen Shifteinheit, einer Hydriereinheit, einer Prereformiereinheit, einer Entschwfelungseinheit, einer Dechlorierungseinheit, einer Quecksilberadsorptionseinheit, einer Methanisierungseinheit oder beliebigen Kombinationen hiervon ausgewählt ist oder sind.

10. Verfahren (200) nach einem der vorstehenden Ansprüche, bei der eine Anzahl der Anlagen oder Anlagenkomponenten (110-150) 2, 3, 4, 5 oder mehr als 5 und insbesondere bis zu 10 beträgt.

11. Verfahren (200) nach einem der vorstehenden Ansprüche, bei dem die Anfahr- und/oder Abfahreinrichtung (10) in aufeinanderfolgenden oder disjunkten Zeiträumen für den Betrieb der Anlagen oder Anlagenkomponenten (110-150) verwendet wird.

12. Anlagenverbund (100) zur Bearbeitung unterschiedlicher Reaktionseinsätze, mit unterschiedlichen Anlagen oder Anlagenkomponenten (110-150), wobei die Anlagen oder Anlagenkomponenten (110-150) jeweils für einen Anfahr- und/oder Abfahrbetrieb unter Einsatz einer Anfahr- und/oder Abfahreinrichtung (10) eingerichtet sind, und wobei für die unterschiedlichen Anlagen oder Anlagenkomponenten (110-150) dieselbe Anfahr- und/oder Abfahreinrichtung (10) bereitgestellt ist.

13. Anlagenverbund nach Anspruch 12, der zur Durchführung eines Verfahrens nach einem der Ansprüche 1 bis 11 eingerichtet ist.
